# EUROPEAN PATENT APPLICATION

(11) **EP 4 643 803 A1**
(43) Date of publication of application: **05.11.2025**
(21) Application number: 24315222.0
(22) Date of filing: 30.04.2024
(51) Int. Cl.: A61B 34/20

(54) **SURGICAL NAVIGATION SYSTEM**

(71) Applicant: MinMaxMedical, 38400 Saint-Martin-d'Hères (FR)
(72) Inventor: LARUE, Jean-François, 38400 Saint-Martin-d'Hères (FR); HUGUEL, Loïc, 38400 Saint-Martin-d'Hères (FR); BOUFFETTE, Céline, 38400 Saint-Martin-d'Hères (FR)
(74) Representative: INNOV-GROUP

(57) **Abstract**

This surgical navigation system can determine a pose for "M" optical markers (M1-M16) among "Nₜₒₜ" optical markers (M1-M16), even if one of the "M" optical markers (M1-M16) is partially or totally hidden in the field of view of one monocular camera (C1-C3) among "N" monocular cameras (C1-C3), N≥3; two conditions must be met:
- 2D images of the number "M" of optical markers (M1-M16) are captured, at a given time, by at least two monocular cameras (C1-C3) of the set, where "M" is an integer satisfying 5≤M≤Nₜₒₜ;
- each monocular camera of the set (C1-C3) captures 2D images, at the given time, of a cumulative number "m" of different optical markers (M1-M16) that are shared with the other "N-1" monocular cameras (C1-C3) of the set, where "m" is an integer satisfying 5≤m≤M.

## Description

### Technical field

The invention relates to the technical field of surgical navigation systems.

The invention is notably applicable to navigate a surgical tool during a surgical intervention.

### Background art

A surgical navigation system known from prior art, notably from US 2016/0225192 A1, comprises a head-mounted display (HMD) which may be connected to more than one camera. Video streams from the cameras can be provided to an operational function that estimates distance to an object viewed by the cameras. The operational function can include triangulation of distance to the object based on angular offset of the object viewed in the video streams and a known distance between the cameras, §0032.

Such a surgical navigation system from prior art is not entirely satisfactory because triangulation cannot be performed in an appropriate manner when trackers (e.g. optical trackers) are totally or partially hidden. Yet, optical trackers are likely to be hidden when something or someone comes in between the cameras and the optical trackers.

### Disclosure of the invention

The invention aims to completely or partly overcome the aforementioned drawbacks. To this end, one subject of the invention is a surgical navigation system, comprising:
- a set of "Nₜₒₜ" optical markers, configured to track poses of objects, where "Nₜₒₜ" is an integer satisfying Nₜₒₜ>5; the "Nₜₒₜ" optical markers of the set having unknown relative positions therebetween and unknown absolute positions;
- a set of "N" monocular cameras, arranged to capture 2D images of the set of "Nₜₒₜ" optical markers, where "N" is an integer satisfying N≥3; the "N" monocular cameras of the set being movable therebetween, the "N" monocular cameras of the set having unknown relative positions therebetween and unknown absolute positions; each monocular camera of the set being movable with respect to the "Nₜₒₜ" optical markers;

2D images of a number "M" of optical markers being captured, at a given time, by at least two monocular cameras of the set, where "M" is an integer satisfying 5≤M≤Nₜₒₜ;
each monocular camera of the set capturing 2D images, at the given time, of a cumulative number "m" of different optical markers that are shared with the other "N-1" monocular cameras of the set, where "m" is an integer satisfying 5≤m≤M;
   - a data processor, configured to perform steps at the given time:
      a) extract, for each monocular camera of the set, 2D coordinates of optical markers whose 2D images are captured by the corresponding monocular camera;
      b) generate all k-tuples of the 2D coordinates extracted in step a), each k-tuple associating a combination of "k" 2D coordinates extracted for "k" monocular cameras of the set, where "k" is an integer satisfying 2≤k≤N;
      c) initialize a pose for each monocular camera of the set in a 3D reference coordinate system;
      d) build 3D points in the 3D reference coordinate system from projection lines of the k-tuples generated in step b);
      e) reproject the 3D points built in step d) onto the 2D images captured by the "N" monocular cameras of the set, and determine a reprojection error for each 3D point;
      f) select the number "M" of 3D points reprojected in step e) that minimize the reprojection error;
      g) determine a pose for each monocular camera of the set in the 3D reference coordinate system that minimizes the reprojection error for the "M" 3D points selected in step f);
      h) compute a pose for the "M" optical markers in the 3D reference coordinate system from the poses determined in step g).

Thus, such a surgical navigation system according to the invention can determine a pose for the "M" optical markers, even if one of the "M" optical markers is partially or totally hidden in the field of view of one monocular camera of the set. To make it happen, two conditions must be met:
(i) 2D images of the number "M" of optical markers are captured, at a given time, by at least two monocular cameras of the set, where "M" is an integer satisfying 5≤M≤Nₜₒₜ;
(ii) each monocular camera of the set captures 2D images, at the given time, of a cumulative number "m" of different optical markers that are shared with the other "N-1" monocular cameras of the set, where "m" is an integer satisfying 5≤m≤M.

Then, such a data processor is able to compute a pose for the "M" optical markers by performing steps a) to h), including when one of the "M" optical markers is partially or totally hidden. However, when M≠Nₜₒₜ, the poses of the "Nₜₒₜ-M" optical markers cannot be computed in so far as 2D images thereof are not captured, at the given time, by at least two monocular cameras of the set.

Therefore, such a surgical navigation system according to the invention makes it possible to track poses of objects, including when one of the "M" optical markers is partially or totally hidden.

The surgical navigation system according to the invention may include one or more of the following features.

According to one feature of the invention, the data processor is configured to perform step g) by:
g₁) refining the pose initialized in step c) for each monocular camera of the set;
gz) executing steps d), e) and f);
g₃) iterating steps g₁) and g₂) until the pose refined in step g₁) minimizes the reprojection error determined in step e).

Thus, one advantage afforded is that of improving the calculation accuracy of a pose for the "M" optical markers.

According to one feature of the invention, the data processor is further configured to perform step g) by:
g₄) comparing the minimized reprojection error at the end of step g₃) with a predetermined threshold;
g₅) executing step g₃) until the minimized reprojection error is inferior to the predetermined threshold.

Thus, one advantage afforded is that of defining a satisfactory safety level for the calculation of a pose for the "M" optical markers in order to improve the reliability thereof.

According to one feature of the invention, the data processor is configured to perform steps c), d), e), f) and g) by implementing a data fusion algorithm, preferably a bundle adjustment algorithm.

Thus, one advantage afforded by the bundle adjustment algorithm is that of providing both the 3D points describing the scene geometry and the poses of the monocular cameras.

According to one feature of the invention, the data fusion algorithm includes a marker assignment algorithm designed to assign 2D coordinates extracted in step a) to an optical marker whose 2D images are captured by the corresponding monocular camera.

Thus, one advantage afforded is that of enhancing the reliability of the fusion data.

According to one feature of the invention, the data processor is configured to perform step c) by generating a random pose for each monocular camera of the set.

According to one feature of the invention, the data processor is configured to perform step c), at a time later than the given time, by initializing the poses with the poses determined in step g) at the given time.

Thus, one advantage afforded is that of reducing the convergence time when performing step g) in comparison with a new random pose for each monocular camera of the set.

According to one feature of the invention, the set of "Nₜₒₜ" optical markers are selected from retro-reflective markers, colored marks, textured marks, geometrical patterns, coded targets, scale bars.

According to one feature of the invention, the surgical navigation system comprises a wireless module configured to connect the data processor to the set of "N" monocular cameras.

Thus, one advantage afforded is that a wireless module avoids cluttering the operation room.

According to one feature of the invention, the surgical navigation system comprises:
- a pair of glasses;
- a head-up display, mounted on the pair of glasses, and connected to the data processor;
wherein a monocular camera of the set is fixedly positioned with respect to the head-up display.

Thus, one advantage afforded is that of displaying useful information (e.g. poses of the tracked objects) for the person equipped with the pair of glasses. One second advantage afforded is that of having the nearest view possible of the scene, without impeding the point of view between the surgeon and the medical operation.

According to one feature of the invention, the surgical navigation system comprises a head-mounted display connected to the data processor, wherein a monocular camera of the set is fixedly positioned with respect to the head-mounted display.

Thus, one advantage afforded is that of displaying useful information (e.g. poses of the tracked objects) for the person equipped with the head-mounted display. One second advantage afforded is that of having the nearest view possible of the scene, without impeding the point of view between the surgeon and the medical operation.

According to one feature of the invention:
- the "M" optical markers are distributed among at least one optical tracker having a known geometry;
- said at least one optical tracker is configured to track poses of objects.

According to one feature of the invention, the data processor is further configured to perform, after step h), steps:
- assign 2D coordinates extracted in step a) to an optical tracker among said at least one optical tracker;
- compute poses of the objects tracked by said at least one optical tracker in the 3D reference coordinate system, from the poses computed in step h).

Thus, one advantage afforded is that of computing poses of tracked objects, including when the "M" optical markers are distributed among several optical trackers.

### Definitions

- The term "object" is understood to mean an object used in surgery. By way of non-limiting examples, the term "object" may refer to a surgical tool (instrument), a bone or an implant.
- The terms "2D" and "3D" are understood to mean 2-dimensional and 3-dimensional respectively.
- The term "2D images of a number "M" of optical markers being captured, at a given time, by at least two monocular cameras of the set" is understood to mean that the "M" optical markers are entirely visible in the 2D images captured by said at least two monocular cameras of the set. In general, the term "capture 2D images of markers by a monocular camera" is understood to mean that said markers are entirely (integrally) visible in the 2D images captured by said monocular camera.
- The term "a cumulative number "m" of different optical markers that are shared with the other "N-1" monocular cameras of the set" is understood to mean that any given monocular camera of the set shares with the other "N-1" monocular cameras of the set a number ("m") of markers that is defined by adding successive occurrences of different markers that are (entirely) visible by both the given monocular camera and each of the other monocular cameras of the set. The term "different markers" is understood to mean that an occurrence is not added when the same marker is redundantly shared by the given monocular camera and at least two of the other monocular cameras of the set. In that case, only one marker is counted in the cumulative number.
- The term "data processor" is understood to mean a device, such a calculator or a computer, that performs operations on data.
- The term "k-tuple" is understood to mean a list of "k" elements, where "k" is an integer satisfying 2≤k≤N; each element of the list being 2D coordinates extracted from a different monocular camera. For example, when N=3, all k-tuples with 2≤k≤3 are generated in step b), that is to say all 2-tuples (doublets) and all 3-tuples (triplets) are generated in step b). In other words, all 2-tuples cover all combinations of two 2D coordinates extracted by two monocular cameras; all 3-tuples cover all combinations of three 2D coordinates extracted by three monocular cameras. Of course, the number of 2D coordinates extracted by each monocular camera of the set may be different, depending on the optical markers whose 2D images are captured by the corresponding monocular camera.
- The term "pose" is understood to mean the position and the spatial orientation. The pose of an entity can be defined by three parameters of position and three parameters of orientation.
- The term "3D reference coordinate system" is understood to mean a geometric reference frame used to describe poses of entities (markers, tracked objects, monocular cameras etc.) that may be obtained by a 3D geometrical reconstruction.

### Brief description of the drawings

Other features and advantages will become apparent in the detailed description of various embodiments of the invention, the description being accompanied by examples and references to the appended drawings.
Figure 1 is a schematic perspective view of a surgical navigation system according to the invention in a surgical room.
Figure 2 is a schematic representation of fields of view belonging to monocular cameras in a surgical navigation system according to the invention, said fields of view capturing certain optical markers at a given time.
Figure 3 is a schematic representation of fields of view belonging to monocular cameras in a surgical navigation system according to the invention, said fields of view capturing a certain number of optical markers at a given time.
Figure 4 is analogous to Figure 3, illustrating a different spatial configuration of fields of view.
Figure 5 is analogous to Figure 4, illustrating another spatial configuration of fields of view.

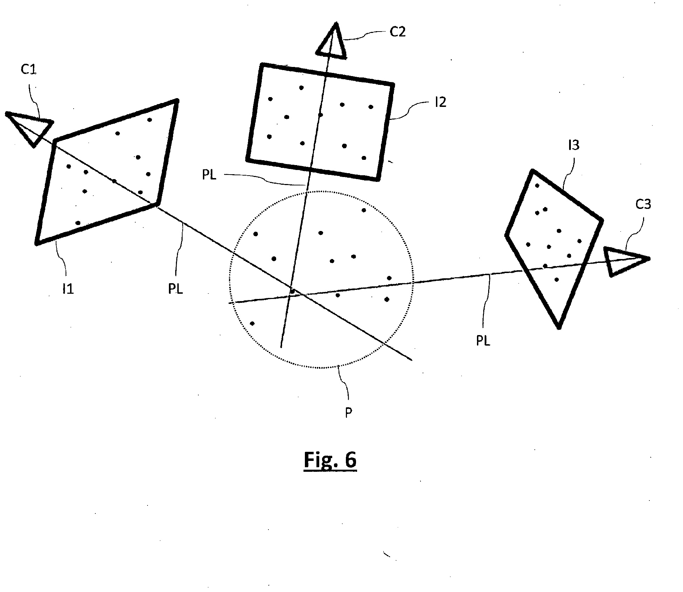

Figure 6 is a schematic perspective view, illustrating 3D points built in step d) and 3D points selected in step f) that minimize the reprojection error.

It should be noted that, for the sake of readability and ease of understanding, the drawings described above are schematic and are not necessarily to scale.

### Detailed description of embodiments

Elements that are identical or provide the same function will carry the same references for the various embodiments, for the sake of simplicity.

One subject of the invention is a surgical navigation system, comprising:
- a set of "Nₜₒₜ" optical markers M1-M16, configured to track poses-of objects, where "Nₜₒₜ" is an integer satisfying Nₜₒₜ>5; the "Nₜₒₜ" optical markers M1-M16 of the set having unknown relative positions therebetween and unknown absolute positions;
- a set of "N" monocular cameras C1-C3, arranged to capture 2D images I1-I3 of the set of "Nₜₒₜ" optical markers M1-M16, where "N" is an integer satisfying N≥3; the "N" monocular cameras C1-C3 of the set being movable therebetween, the "N" monocular cameras C1-C3 of the set having unknown relative positions therebetween and unknown absolute positions; each monocular camera C1-C3 of the set being movable with respect to the "Nₜₒₜ" optical markers M1-M16;
   2D images I1-I3 of a number "M" of optical markers M1-M16 being captured, at a given time, by at least two monocular cameras C1-C3 of the set, where "M" is an integer satisfying 5≤M≤Nₜₒₜ;
   each monocular camera C1-C3 of the set capturing 2D images I1-I3, at the given time, of a cumulative number "m" of different optical markers M1-M16 that are shared with the other "N-1" monocular cameras C1-C3 of the set, where "m" is an integer satisfying 5≤m≤M;
- a data processor DP, configured to perform steps at the given time:
   a) extract, for each monocular camera C1-C3 of the set, 2D coordinates of optical markers M1-M16 whose 2D images I1-I3 are captured by the corresponding monocular camera C1-C3;
   b) generate all k-tuples of the 2D coordinates extracted in step a), each k-tuple associating a combination of "k" 2D coordinates extracted for "k" monocular cameras C1-C3 of the set, where "k" is an integer satisfying 2≤k≤N;
   c) initialize a pose for each monocular camera C1-C3 of the set in a 3D reference coordinate system;
   d) build 3D points P in the 3D reference coordinate system from projection lines PL of the k-tuples generated in step b);
   e) reproject the 3D points P built in step d) onto the 2D images I1-I3 captured by the "N" monocular cameras C1-C3 of the set, and determine a reprojection error for each 3D point P;
   f) select the number "M" of 3D points P reprojected in step e) that minimize the reprojection error;
   g) determine a pose for each monocular camera C1-C3 of the set in the 3D reference coordinate system that minimizes the reprojection error for the "M" 3D points P selected in step f);
   h) compute a pose for the "M" optical markers M1-M16 in the 3D reference coordinate system from the poses determined in step g).

### Optical markers

The surgical navigation system comprises a set of "Nₜₒₜ" optical markers M1-M16, configured to track poses of objects. "Nₜₒₜ" is an integer satisfying Nₜₒₜ>5. In other words, optical markers M1-M16 (also known as fiducial markers) are used to locate the position and orientation of objects in combination with the set of monocular cameras C1-C3. The "Nₜₒₜ" optical markers M1-M16 of the set have unknown relative positions therebetween, and unknown absolute positions.

The set of "Nₜₒₜ" optical markers M1-M16 are advantageously selected from retro-reflective markers, colored marks, textured marks, geometrical patterns, coded targets, scale bars.

The set of "Nₜₒₜ" optical markers M1-M16 may be distributed among at least one optical tracker T, generally among several optical trackers T, in order to track poses of objects.

### Monocular cameras

The set of "N" monocular cameras C1-C3 is arranged to capture 2D images I1-I3 of the set of "Nₜₒₜ" optical markers M1-M16. "N" is an integer satisfying N≥3. The "N" monocular cameras C1-C3 of the set are movable therebetween. The "N" monocular cameras C1-C3 of the set have unknown relative positions therebetween and unknown absolute positions. Each monocular camera C1-C3 of the set is movable with respect to the "Nₜₒₜ" optical markers M1-M16.

2D images I1-I3 of a number "M" of optical markers M1-M16 are captured, at a given time, by at least two monocular cameras C1-C3 of the set. "M" is an integer satisfying 5≤M≤Nₜₒₜ. The "M" optical markers may be distributed among at least one optical tracker T, generally among several optical trackers T. Said at least one optical tracker T is configured to track poses of objects.

Each monocular camera C1-C3 of the set captures 2D images I1-I3, at the given time, of a cumulative number "m" of different optical markers M1-M16 that are shared with the other "N-1" monocular cameras C1-C3 of the set. "m" is an integer satisfying 5≤m≤M.

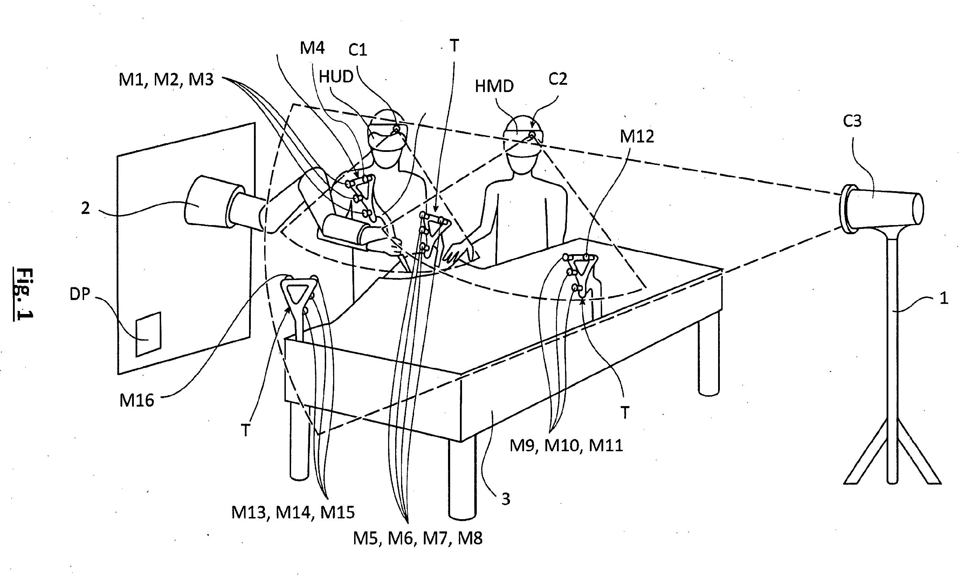

Each monocular camera C1-C3 of the set may comprise a light source configured to emit infrared radiations. The light source may include a set of light-emitting diodes. The light source is advantageously integrated in the corresponding monocular camera C1-C3 for compactness reasons. As illustrated in figure 1, a monocular camera C1-C3 of the set may be mounted on an autonomous support 1.

### Data processor: step a)

The data processor DP is configured to extract, for each monocular camera C1-C3 of the set, 2D coordinates of optical markers M1-M16 whose 2D images I1-I3 are captured by the corresponding monocular camera C1-C3.

Various techniques and algorithms known by a person skilled in the art may be employed to perform this step. These techniques may include edge detection algorithms (like Sobel or Canny), corner detection algorithms (like Harris corner detector), blob detection algorithms (like the Laplacian of Gaussian).

### Data processor: step b)

The data processor DP is configured to generate all k-tuples of the 2D coordinates extracted in step a). Each k-tuple associates a combination of "k" 2D coordinates extracted for "k" monocular cameras C1-C3 of the set, where "k" is an integer satisfying 2≤k≤N.

For example, when N=3, all k-tuples with 2≤k≤3 are generated in step b), that is to say all 2-tuples (doublets) and all 3-tuples (triplets) are generated in step b). All 2-tuples cover all combinations of two 2D coordinates extracted by two monocular cameras C1-C3. All 3-tuples cover all combinations of three 2D coordinates extracted by three monocular cameras C1-C3. As a pure illustrative (non-functional) example, let us assume that:
- 2D coordinates "c11" of one optical marker are extracted for monocular camera C1,
- 2D coordinates "c21, c22" of two optical markers are extracted for monocular camera C2,
- 2D coordinates "c31, c32, c33" of three optical markers are extracted for monocular camera C3.

Then, {[c11, c21], [c11, c22], [c11, c31], [c11, c32], [c11, c33], [c21, c31], [c21, c32], [c21, c33], [c22, c31], [c22, c32], [c22, c33]} are all eleven 2-tuples generated in step b). In addition, {[c11, c21, c31], [c11, c21, c32], [c11, c21, c33], [c11, c22, c31], [c11, c22, c32], [c11, c22, c33]} are all six 3-tuples generated in step b).

To this end, the data processor is configured to store the 2D coordinates extracted in step a) in an array, a list, or another data structure.

### Data processor: step c)

The data processor DP is configured to initialize a pose for each monocular camera C1-C3 of the set in a 3D reference coordinate system at the given time. The data processor DP is advantageously configured to perform step c) at the given time by generating a random pose for each monocular camera C1-C3 of the set.

The data processor DP is advantageously configured to perform step c), at a time later than the given time, by initializing the poses with the poses determined in step g) at the given time.

The data processor DP is advantageously configured to perform step c) by implementing a data fusion algorithm, preferably a bundle adjustment algorithm. Examples of implementations of a bundle adjustment algorithm can be found in the document (PhD thesis): E. Mouragnon, "Reconstruction 3D et localisation simultanée de cameras mobiles : une approche temps-réel par ajustement de faisceaux local", 2007, or in the document R. Li et al., "Incremental bundle adjustment techniques using networked overhead and ground imagery for long-range autonomous Mars Rover localization", Proc. of the 8th International Symposium on Artifical Intelligence, Robotics and Automation in Space - iSAIRAS, 2005.

### Data processor: step d)

The data processor DP is configured to build 3D points P in the 3D reference coordinate system from projection lines PL of the k-tuples generated in step b).

The data processor DP is advantageously configured to perform step d) by implementing a data fusion algorithm, preferably a bundle adjustment algorithm.

### Data processor: step e)

The data processor DP is configured to:
- reproject the 3D points P built in step d) onto the 2D images 11-13 captured by the "N" monocular cameras C1-C3 of the set, and
- determine a reprojection error for each 3D point P.

The data processor DP is advantageously configured to perform step e) by implementing a data fusion algorithm, preferably a bundle adjustment algorithm.

### Data processor: step f)

The data processor DP is configured to select the number "M" of 3D points P reprojected in step e) that minimize the reprojection error. To this end, a pixel threshold is advantageously defined to improve the reliability of the selection.

The number "M" of 3D points P minimizing the reprojection error corresponds to the number "M" of optical markers M1-M16 whose 2D images I1-I3 are captured, at the given time, by at least two monocular cameras C1-C3 of the set.

The data processor DP is advantageously configured to perform step f) by implementing a data fusion algorithm, preferably a bundle adjustment algorithm.

### Data processor: step g)

The data processor DP is configured to determine a pose for each monocular camera C1-C3 of the set in the 3D reference coordinate system that minimizes the reprojection error for the "M" 3D points P selected in step f).

The data processor DP is advantageously configured to perform step g) by:
g₁) refining the pose initialized in step c) for each monocular camera C1-C3 of the set;
gz) executing steps d), e) and f);
g₃) iterating steps g₁) and g₂) until the pose refined in step g₁) minimizes the reprojection error determined in step e).

The data processor DP is further advantageously configured to perform step g) by:
g₄) comparing the minimized reprojection error at the end of step g₃) with a predetermined threshold;
g₅) executing step g₃) until the minimized reprojection error is inferior to the predetermined threshold.

The data processor DP is advantageously configured to perform step g) by implementing a data fusion algorithm, preferably a bundle adjustment algorithm. The data fusion algorithm advantageously includes a marker assignment algorithm designed to assign 2D coordinates extracted in step a) to an optical marker M1-M16 whose 2D images I1-I3 are captured by the corresponding monocular camera C1-C3. Various techniques and algorithms known by a person skilled in the art may be employed as a marker assignment algorithm. By way of non-limiting examples, the Nearest Neighbor Assignment, the Joint Probabilistic Data Association (JPDA), the Multiple Hypothesis Tracking (MHT), the Global Nearest Neighbor (GNN), the Probabilistic Data Association Filter (PDAF) may be employed as marker assignment algorithms.

### Data processor: step h)

The data processor DP is configured to compute a pose for the "M" optical markers M1-M16 in the 3D reference coordinate system from the poses determined in step g). The data processor may be configured to perform step h) by using a matrix transformation between coordinate systems known by a skilled person in the art. The matrix transformation may be a rigid transformation referring to a geometric transformation of a Euclidean space that preserves the distance between every pair of points. The rigid transformation may include rotations, translations, reflections, or a combination thereof. By way of a non-limiting example, the data processor DP may implement Kabsch-Umeyama algorithm to perform step h). Other algorithms (Iterative Closest Point ICP, Random Sample Consensus RANSAC) are also conceivable.

The "M" optical markers may be distributed among at least one optical tracker T having a known geometry, generally among several optical trackers T. Said at least one optical tracker T is configured to track poses of objects. When the "M" optical markers M1-M16 are distributed among at least one optical tracker T having a known geometry, the data processor DP is further advantageously configured to perform, after step h), steps:
- assign 2D coordinates extracted in step a) to an optical tracker T among said at least one optical tracker T, by implementing a marker assignment algorithm;
- compute poses of objects tracked by said at least one optical tracker T in the 3D reference coordinate system, from the poses computed in step h), by using a matrix transformation between coordinate systems.

It should be noted that knowledge of the geometry of said at least one optical tracker T is not a piece of information used when performing steps a) to h).

### Equipment

The surgical navigation system advantageously comprises a wireless module (not illustrated) configured to connect the data processor DP to the set of "N" monocular cameras C1-C3.

According to one embodiment, the surgical navigation system comprises:
- a pair of glasses;
- a head-up display HUD, mounted on the pair of glasses, and connected to the data processor DP.

A monocular camera C1-C3 of the set is fixedly positioned with respect to the head-up display HUD. Said monocular camera is advantageously mounted on the eyeglass frame of the pair of glasses, preferably by clipping (snap-fit assembly).

According to another embodiment, the surgical navigation comprises a head-mounted display HMD connected to the data processor DP. A monocular camera C1-C3 of the set is fixedly positioned with respect to the head-mounted display HMD.

The surgical navigation system may be associated with a robotic arm 2 for assisting a user (e.g. a surgeon) during a surgical intervention when a patient lies on an operating table 3.

### Example n°1

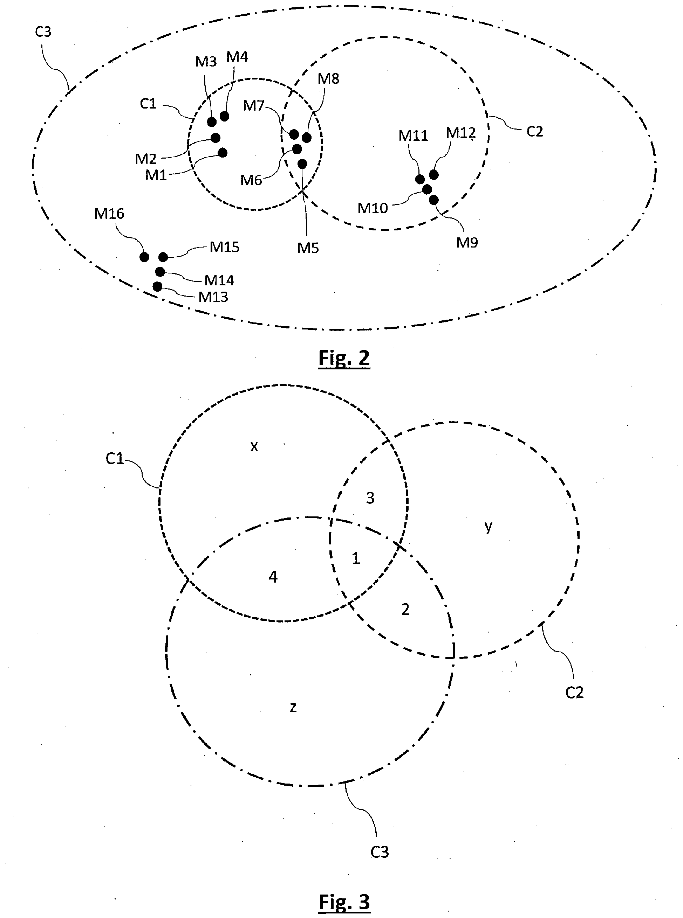

As illustrated in figures 1 and 2, Nₜₒₜ=16 and N=3.

Four optical markers M13-M16 are only seen by the monocular camera C3.

2D images of twelve markers M1-M12 are captured, at a given time, by at least two monocular cameras C1-C3 of the set: M=12. Indeed, 2D images of four optical markers M1-M4 are captured by only two monocular cameras C1, C3. 2D images of four optical markers M5-M8 are captured by all monocular cameras C1, C2, C3. 2D images of four optical markers M9-M12 are captured by only two monocular cameras C2, C3.

Each monocular camera C1-C3 of the set captures 2D images, at the given time, of a cumulative number "m" of different optical markers that are shared with the other two monocular cameras of the set, where "m" is an integer satisfying 5≤m≤M.

For monocular camera C1: m=m1=4 [M5-M8 shared with monocular camera C2] + 8 [M1-M8 shared with monocular camera C3] - 4 [M5-M8 are redundant] = 8.

For monocular camera C2: m=m2=4 [M5-M8 shared with monocular camera C1] + 8 [M5-M12 shared with monocular camera C3] - 4 [M5-M8 are redundant] = 8.

For monocular camera C3: m=m3=8 [M1-M8 shared with monocular camera C1] + 8 [M5-M12 shared with monocular camera C2] - 4 [M5-M8 are redundant] = 12.

### Example n°2

As illustrated in figure 3, Nₜₒₜ=x+y+z+10 and N=3. "x" is the number of optical markers only seen by the monocular camera C1. "y" is the number of optical markers only seen by the monocular camera C2. "z" is the number of optical markers only seen by the monocular camera C3.

2D images of ten markers (4+3+1+2) are captured, at a given time, by at least two monocular cameras C1-C3 of the set: M=10. Indeed, 2D images of four optical markers are captured by only two monocular cameras C1, C3. 2D images of three optical markers are captured by only two monocular cameras C1, C2. 2D images of one optical marker are captured by all monocular cameras C1, C2, C3. 2D images of two optical markers are captured by only two monocular cameras C2, C3.

Each monocular camera C1-C3 of the set captures 2D images, at the given time, of a cumulative number "m" of different optical markers that are shared with the other two monocular cameras of the set, where "m" is an integer satisfying 5≤m≤M.

For monocular camera C1: m=m1=4 [shared with monocular camera C2] + 5 [shared with monocular camera C3] - 1 [shared with both monocular cameras C2, C3: redundant] = 8.

For monocular camera C2: m=m2=4 [shared with monocular camera C1] + 3 [shared with monocular camera C3] - 1 [shared with both monocular cameras C1, C3: redundant] = 6.

For monocular camera C3: m=m3=5 [shared with monocular camera C1] + 3 [shared with monocular camera C2] - 1 [shared with both monocular cameras C1, C2: redundant] = 7.

### Example n°3

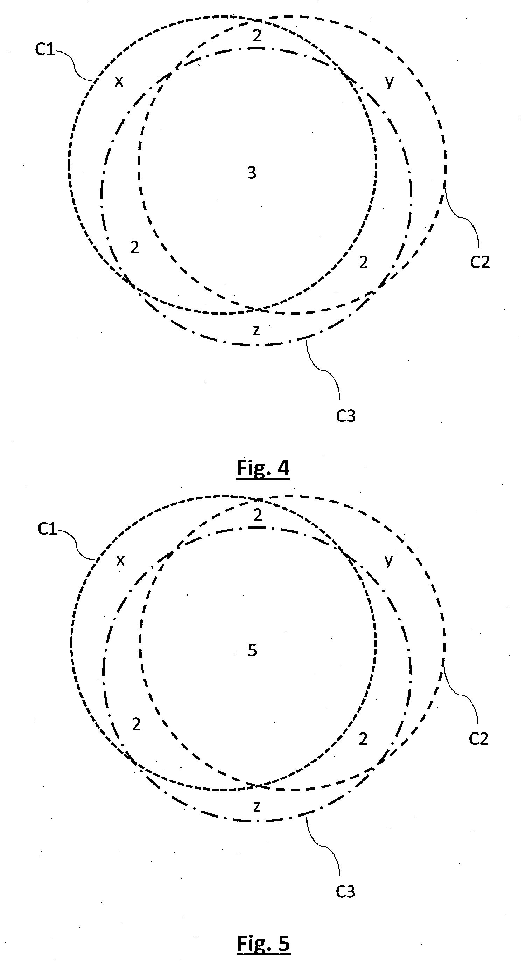

As illustrated in figure 4, Nₜₒₜ=x+y+z+9 and N=3. "x" is the number of optical markers only seen by the monocular camera C1. "y" is the number of optical markers only seen by the monocular camera C2. "z" is the number of optical markers only seen by the monocular camera C3.

2D images of nine markers (2+2+3+2) are captured, at a given time, by at least two monocular cameras C1-C3 of the set: M=9. Indeed, 2D images of two optical markers are captured by only two monocular cameras C1, C3. 2D images of two optical markers are captured by only two monocular cameras C1, C2. 2D images of three optical markers are captured by all monocular cameras C1, C2, C3. 2D images of two optical markers are captured by only two monocular cameras C2, C3.

Each monocular camera C1-C3 of the set captures 2D images, at the given time, of a cumulative number "m" of different optical markers that are shared with the other two monocular cameras of the set, where "m" is an integer satisfying 5≤m≤M.

For monocular camera C1: m=m1=5 [shared with monocular camera C2] + 5 [shared with monocular camera C3] - 3 [shared with both monocular cameras C2, C3: redundant] = 7.

For monocular camera C2: m=m2=5 [shared with monocular camera C1] + 5 [shared with monocular camera C3] - 3 [shared with both monocular cameras C1, C3: redundant] = 7.

For monocular camera C3: m=m3=5 [shared with monocular camera C1] + 5 [shared with monocular camera C2] - 3 [shared with both monocular cameras C1, C2: redundant] = 7.

The two optical markers captured by the monocular cameras C1, C2 (or by the monocular cameras C1, C3, or by the monocular cameras C2, C3) can be hidden without affecting the ability to compute the pose for the "M" optical markers in step h). In that case, M=9-2=7; m1=m2=7-2=5; m3=7.

### Example n°4

As illustrated in figure 5, Nₜₒₜ=x+y+z+11 and N=3. "x" is the number of optical markers only seen by the monocular camera C1. "y" is the number of optical markers only seen by the monocular camera C2. "z" is the number of optical markers only seen by the monocular camera C3.

2D images of eleven markers (2+2+5+2) are captured, at a given time, by at least two monocular cameras C1-C3 of the set: M=11. Indeed, 2D images of two optical markers are captured by only two monocular cameras C1, C3. 2D images of two optical markers are captured by only two monocular cameras C1, C2. 2D images of five optical markers are captured by all monocular cameras C1, C2, C3. 2D images of two optical markers are captured by only two monocular cameras C2, C3.

Each monocular camera C1-C3 of the set captures 2D images, at the given time, of a cumulative number "m" of different optical markers that are shared with the other two monocular cameras of the set, where "m" is an integer satisfying 5≤m≤M.

For monocular camera C1: m=m1=7 [shared with monocular camera C2] + 7 [shared with monocular camera C3] - 5 [shared with both monocular cameras C2, C3: redundant] = 9.

For monocular camera C2: m=m2=7 [shared with monocular camera C1] + 7 [shared with monocular camera C3] - 5 [shared with both monocular cameras C1, C3: redundant] = 9.

For monocular camera C3: m=m3=7 [shared with monocular camera C1] + 7 [shared with monocular camera C2] - 5 [shared with both monocular cameras C1, C2: redundant] = 9.

Any monocular camera C1-C3 can be hidden (losing four optical markers) without affecting the ability to compute the pose for the "M" optical markers in step h). In that case, assuming the monocular camera C1 being hidden, M=11-4=7; m1=9-4=5; m2=m3=9.

The invention is not limited to the described embodiments. A person skilled in the art is capable of considering all technically feasible combinations thereof and of substituting them with equivalents.

## Claims

1. A surgical navigation system, comprising:
- a set of "Nₜₒₜ" optical markers (M1-M16), configured to track poses of objects, where "Nₜₒₜ" is an integer satisfying Nₜₒₜ>5; the "Nₜₒₜ" optical markers (M1-M16) of the set having unknown relative positions therebetween and unknown absolute positions;
- a set of "N" monocular cameras (C1-C3), arranged to capture 2D images (I1-I3) of the set of "Nₜₒₜ" optical markers (M1-M16), where "N" is an integer satisfying N≥3; the "N" monocular cameras (C1-C3) of the set being movable therebetween, the "N" monocular cameras (C1-C3) of the set having unknown relative positions therebetween and unknown absolute positions; each monocular camera (C1-C3) of the set being movable with respect to the "Nₜₒₜ" optical markers (M1-M16);
2D images (I1-I3) of a number "M" of optical markers (M1-M16) being captured, at a given time, by at least two monocular cameras (C1-C3) of the set, where "M" is an integer satisfying 5≤M≤Nₜₒₜ;
each monocular camera (C1-C3) of the set capturing 2D images (I1-I3), at the given time, of a cumulative number "m" of different optical markers (M1-M16) that are shared with the other "N-1" monocular cameras (C1-C3) of the set, where "m" is an integer satisfying 5≤m≤M;
- a data processor (DP), configured to perform steps at the given time:
a) extract, for each monocular camera (C1-C3) of the set, 2D coordinates of optical markers (M1-M16) whose 2D images (I1-I3) are captured by the corresponding monocular camera (C1-C3);
b) generate all k-tuples of the 2D coordinates extracted in step a), each k-tuple associating a combination of "k" 2D coordinates extracted for "k" monocular cameras (C1-C3) of the set, where "k" is an integer satisfying 2≤k≤N;
c) initialize a pose for each monocular camera (C1-C3) of the set in a 3D reference coordinate system;
d) build 3D points (P) in the 3D reference coordinate system from projection lines (PL) of the k-tuples generated in step b);
e) reproject the 3D points (P) built in step d) onto the 2D images (I1-I3) captured by the "N" monocular cameras (C1-C3) of the set, and determine a reprojection error for each 3D point (P);
f) select the number "M" of 3D points (P) reprojected in step e) that minimize the reprojection error;
g) determine a pose for each monocular camera (C1-C3) of the set in the 3D reference coordinate system that minimizes the reprojection error for the "M" 3D points (P) selected in step f);
h) compute a pose for the "M" optical markers (M1-M16) in the 3D reference coordinate system from the poses determined in step g).

2. The surgical navigation system according to claim 1, wherein the data processor (DP) is configured to perform step g) by:
g₁) refining the pose initialized in step c) for each monocular camera (C1-C3) of the set;
g₂) executing steps d), e) and f);
g₃) iterating steps g₁) and g₂) until the pose refined in step g₁) minimizes the reprojection error determined in step e).

3. The surgical navigation system according to claim 2, wherein the data processor (DP) is further configured to perform step g) by:
g₄) comparing the minimized reprojection error at the end of step g₃) with a predetermined threshold;
g₅) executing step g₃) until the minimized reprojection error is inferior to the predetermined threshold.

4. The surgical navigation system according to any claim 1 to 3, wherein the data processor (DP) is configured to perform steps c), d), e), f) and g) by implementing a data fusion algorithm, preferably a bundle adjustment algorithm.

5. The surgical navigation system according to claim 4, wherein the data fusion algorithm includes a marker assignment algorithm designed to assign 2D coordinates extracted in step a) to an optical marker (M1-M16) whose 2D images (I1-I3) are captured by the corresponding monocular camera (C1-C3).

6. The surgical navigation system according to any claim 1 to 5, wherein the data processor (DP) is configured to perform step c) by generating a random pose for each monocular camera (G1-C3) of the set.

7. The surgical navigation system according to any claim 1 to 6, wherein the data processor (DP) is configured to perform step c), at a time later than the given time, by initializing the poses with the poses determined in step g) at the given time.

8. The surgical navigation system according to any claim 1 to 7, wherein the set of "Nₜₒₜ" optical markers (M1-M16) are selected from retro-reflective markers, colored marks, textured marks, geometrical patterns, coded targets, scale bars.

9. The surgical navigation system according to any claim 1 to 8, comprising a wireless module configured to connect the data processor (DP) to the set of "N" monocular cameras (C1-C3).

10. The surgical navigation system according to any claim 1 to 9, comprising:
- a pair of glasses;
- a head-up display (HUD), mounted on the pair of glasses, and connected to the data processor (DP);
wherein a monocular camera (C1-C3) of the set is fixedly positioned with respect to the head-up display (HUD).

11. The surgical navigation system according to any claim 1 to 10, comprising a head-mounted display (HMD) connected to the data processor (DP), wherein a monocular camera I (C1-C3) of the set is fixedly positioned with respect to the head-mounted display (HMD).

12. The surgical navigation system according to any claim 1 to 11, wherein:
- the "M" optical markers (M1-M16) are distributed among at least one optical tracker, (T) having a known geometry;
- said at least one optical tracker (T) is configured to track poses of objects.

13. The surgical navigation system according to claim 12, wherein the data processor (DP) is further configured to perform, after step h), steps:
- assign 2D coordinates extracted in step a) to an optical tracker (T) among said at least one optical tracker (T);
- compute poses of the objects tracked by said at least one optical tracker (T) in the 3D reference coordinate system, from the poses computed in step h).
